(19) Europäisches Patentamt
European Patent Office
Office européen des brevets

(11) Veröffentlichungsnummer: **0 256 258**
**A2**

# (12) EUROPÄISCHE PATENTANMELDUNG

(21) Anmeldenummer: **87109105.4**

(22) Anmeldetag: **24.06.87**

(51) Int. Cl.4: **C07C 101/453**, C07C 93/14, A61K 31/205

Patentansprüche für folgenden Vertragsstaaten: AT + ES + GR.

Die Bezeichnung der Erfindung wurde geändert (Richtlinien für die Prüfung im EPA, A-III, 7.3).

(30) Priorität: **10.07.86 DE 3623193**

(43) Veröffentlichungstag der Anmeldung: **24.02.88 Patentblatt 88/08**

(84) Benannte Vertragsstaaten: **AT BE CH DE ES FR GB GR IT LI LU NL SE**

(71) Anmelder: **Grünenthal GmbH**
**Zieglerstrasse 6**
**D-5100 Aachen(DE)**

(72) Erfinder: **Frankus, Ernst, Dr.**
**Höhenkreuzweg 19**
**D-5190 Stolberg(DE)**
Erfinder: **Schmidt-Böthelt, Eberhard**
**Am Widtmannschacht 25**
**D-5190 Stolberg(DE)**
Erfinder: **Winter, Werner, Prof. Dr.**
**Birkenstrasse 5**
**D-5100 Aachen(DE)**

## (54) Salze von Methoxyphenyl-Dimethylaminomethyl-Cyclohexanolen diese enthaltende Arzneimittel und Verfahren zu deren Herstellung.

(57) Es werden als neue, lang anhaltend pharmakologisch wirkende Verbindungen (±)-[1e(m-Methoxyphenyl)-2e-dimethylaminomethyl-cyclohexan-1(a)-ol] - [2-(2',6'-dichloranilino)-phenylacetat] und dessen optisch aktive Formen sowie diese Verbindungen enthaltende Arzneimittel zur Verfügung gestellt. Die Herstellung dieser Verbindungen erfolgt in an sich bekannter Weise durch Umsetzung eines le-(m-Methoxyphenyl)-2e-dimethylaminomethyl-cyclohexan-1(a)-ols oder eines von dessen Salzen mit 2-(2',6'-Dichloranilino)-phenylessigsäure bzw. einem von deren Salzen in Gegenwart eines Reaktionsmediums, in dem die jeweiligen Reaktionspartner löslich sind.

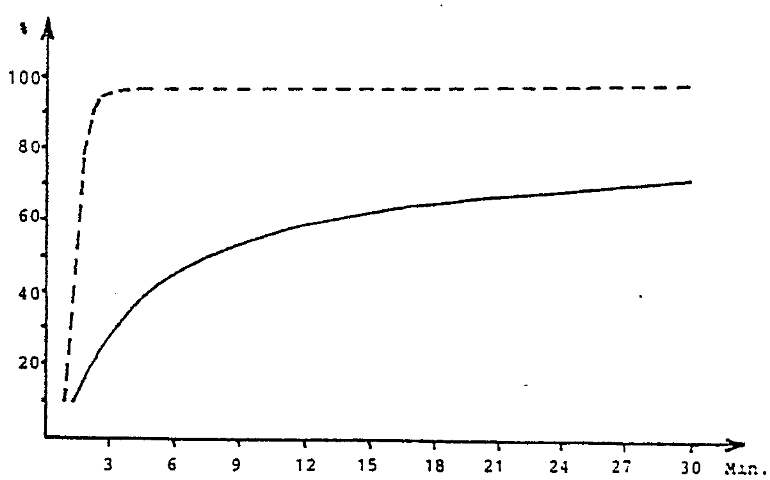


—— (±)-III

– – – I-HCl

Fig. 1

EP 0 256 258 A2

Xerox Copy Centre

**Neue Verbindungen, diese enthaltende Arzneimittel und Verfahren zu deren Herstellung.**

Durch die vorliegende Erfindung werden neue, insbesondere analgetisch und antiphlogistisch wirkende Verbindungen, die aufgrund ihrer Löslichkeitsverhältnisse nach Verabreichung an einen Patienten für längere Zeit einen nahezu konstanten Wirkstoffspiegel im Serum liefern und diese enthaltende Arzneimittel zur Verfügung gestellt. Bei diesen Verbindungen handelt es sich um die aus äquimolaren Mengen 1e-(m-Methoxyphenyl)-2e-dimethylaminomethyl-cyclohexan-1-(a)-ol (I) (als Racemat oder in Form eines der optischen Isomeren)

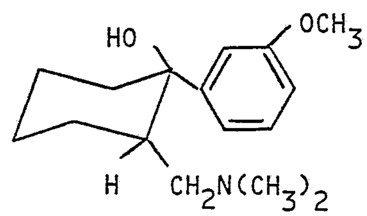

I

und 2-(2',6'-Dichloranilino)-phenylessigsäure (II)

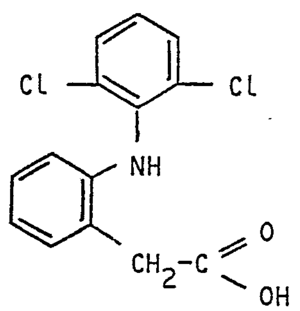

II

erhältlichen salzartigen Produkte (III).

Durch Röntgenstrukturanalyse konnte gezeigt werden, daß in diesen neuen Verbindungen (III) je 2 Moleküle (I) und (II) außer im Sinne der Salzbildung zwischen der Dimethylaminoethylgruppe aus (I) und der Carboxygruppe aus (II) auch über Wasserstoffbrücken komplexartig unter Ausbildung eines hydrophoben Clusters zusammentreten.

(I) und einige von dessen Salzen wie z.B. das Hydrochlorid sind u.a. aus der deutschen Patentschrift 1 199 764 bekannt. Diese Salze von I sind gut wirkende Analgetica mit geringer Toxizität. Das I-Hydrochlorid ist in Wasser bei 20°C zu 29,8 g/100 ml löslich und eignet sich daher insbesondere zur Herstellung von Injektionslösungen oder auch anderer Zubereitungsformen, die dank der guten Löslichkeit des Wirkstoffes einen schnellen Wirkungseintritt (z.B. innerhalb von 30 Minuten) gewährleisten. Die Dosierung von I-Hydrochlorid beträgt für die Einzeldosis im allgemeinen 50 - 100 mg.

(II) und einige Salze, insbesondere Alkalimetallsalze, dieser Säure sind z.B. aus den deutschen Patentschriften 1 543 639 und 1 793 592 bekannt und als insbesondere antiphlogistisch wirkende Substanzen beschrieben worden. Das Natriumsalz besitzt eine Löslichkeit von etwa 3,0 g/100 ml Wasser, und es kann daher z.B. aus Wasser umkristallisiert werden. Im Handel befindliche Injektionslösungen des Natriumsalzes enthalten u.a. 20 % Propylenglycol. Für Erwachsene beträgt die Einzeldosis etwa 50 - 100 mg des II-Natriumsalzes.

Von der neuen Verbindung (±)-III sind bei 20°C in Wasser nur etwa 0,05 g/100 ml löslich, wobei aufgrund der Ausbildung des oben bereits erwähnten hydrophoben Clusters das Auflösen der Substanz langsam und nahezu gleichförmig erfolgt. Unter den in der USP XXI (Seiten 1243 - 1244; Apparat 2) für die Bestimmung der Lösungsgeschwindigkeit von Tabletten, Dragees bzw. Kapseln beschriebenen Bedingungen wurden jeweils für ca. 50 mg (genau gewogen) fein gepulvertes (±)-III bzw. I-Hydrochlorid bei einer Temperatur von 37°C und einer Rührgeschwindigkeit von 100 Umdrehungen/Minute die Lösungsgeschwindigkeiten bei pH 7,2 in 500 ml künstlichem Darmsaft gemessen. Während I-Hydrochlorid bereits nach 2 Minuten zu über 97% gelöst war, waren von (±)-III nach 2 Minuten erst 15,7%, nach 30 Minuten erst 73,7% in Lösung gegangen. Die ermittelten Löslichkeitskurven sind in der Abbildung 1 dargestellt.

Ähnliche Löslichkeitseigenschaften wurden auch für (+)-III bzw. (-)-III ermittelt. Damit eignen sich diese Verbindungen (III) insbesondere zur Herstellung von solchen therapeutischen Zubereitungsformen, die - ohne daß dazu besondere pharmazeutischtechnische Hilfsmaßnahmen ergriffen werden müssen - eine gleichförmige Wirkstoffversorgung des Patienten über einen längeren Zeitraum erlauben.

Die Herstellung der III-enthaltenden Arzneimittel, insbesondere Tabletten, Dragees, Säfte bzw. Sirupe sowie Suppositorien erfolgt in an sich bekannter Weise unter Verwendung anerkannter gebräuchlicher Hilfsstoffe. Als Einzeldosis enthalten diese Zubereitungsformen im allgemeinen etwa 50 bis 250 mg III, insbesondere für eine Dauertherapie kommen aber auch Tabletten, Dragees oder Suppositorien mit einem Wirkstoffgehalt von 500 oder mehr mg III pro Einzelform in Betracht.

Zweckmäßig wird den genannten Zubereitungsformen (speziell den nicht für eine Dauertherapie oder den nur zu Einleitung einer Dauertherapie bestimmten) zur Erzielung eines schnellen Anfangseffektes eine geeignete Menge z.B. von I-Hydrochlorid beigefügt. Bei der Herstellung von Tabletten oder Dragees kann man dabei auch so vorgehen, daß man als Kern einen III enthaltenden Formkörper in an sich bekannter Weise herstellt und diesen dann mit einer I-Hydrochlorid enthaltenden Schicht überzieht.

Die Herstellung von III erfolgt in für die Bildung salzartiger Verbindungen üblicher Weise durch Neutralisation oder durch doppelte Umsetzung zwischen bekannten löslichen Salzen von I (insbesondere den Hydrohalogeniden, vorzugsweise dem Hydrochlorid) als Razemat oder in optisch aktiver Form bzw. von II (insbesondere den Alkali-oder Ammoniumsalzen, vorzugsweise dem Natriumsalz) in Gegenwart geeigneter Lösungsmittel. Dabei kann man in an sich bekannter Weise das Lösungsmittel so auswählen, daß das Produkt (III) aus dem Reaktionsgemisch ausfällt oder durch Abkühlen und/oder Zugabe von mit dem Reaktionsgemisch mischbaren, das Produkt aber nicht lösenden Stoffen, ausgefällt wird. Alternativ kann das Produkt aber auch aus dem Reaktionsgemisch extrahiert und dann aus dem Extrakt isoliert werden.

Die folgenden Beispiele (in denen alle Temperaturangaben unkorrigiert sind) dienen zur weiteren Erläuterung der Erfindung.

Beispiel 1

Zu einer auf 25° - 30°C erwärmten Lösung von 29,6 g 2-(2',6'-Dichloranilino)-phenylessigsäure (II) in 900 ml Diethylether gibt man unter Rühren langsam tropfenweise eine Lösung von 26,3 g (±)-1e(m-Methoxyphenyl)-2e-dimethylaminoethyl-cyclohexan-1(a)-ol [=(±)-I]. Man kühlt dann ab, läßt über Nacht bei 0° - 4°C stehen und filtriert den ausgefallenen Niederschlag ab. Dieser wird mit Diethylether gewaschen und dann getrocknet, wobei man 48 g (= 85% der Theorie) (±)-[1e-(m-Methoxyphenyl)-2e-dimethylaminomethylcyclohexan-1(a)-ol] - [2-(2', 6'-dichloranilino)-phenylacetat] =(±)III in Form farbloser Kristalle erhält. Schmelzpunkt: 151° - 152°C.

Beispiel 2

31,8 g des Natriumsalzes von II werden unter Rühren bei 90° - 95°C in 150 ml Wasser gelöst. Zu dieser Lösung gibt man unter kräftigem Rühren langsam tropfenweise eine Lösung von 30 g (±)-I-Hydrochlorid in 100 ml Wasser. Unter weiterem Rührem kühlt man die entstandene Kristallsuspension auf Raumtemperatur ab, filtriert den Niederschlag ab, wäscht mit Wasser und trocknet das Produkt. Man erhält so 54 g (= 97% der Theorie) (±)-III in Form farbloser Kristalle vom Schmelzpunkt 151° - 152°C.

Beispiel 3

Bei 60°C löst man in einem Gemisch aus je 65 ml Wasser und Ethanol 31,8 g II-Natriumsalz. Unter starkem Rühren gibt man langsam tropfenweise eine Lösung von 30 g (±)-I-Hydrochlorid in 100 ml Wasser zu, kühlt die entstandene Kristallsuspension unter Rühren auf Raumtemperatur ab und läßt sie dann über Nacht im Kühlschrank stehen. Der Niederschlag wird abfiltriert, mit einer Mischung aus 65 ml Ethanol und 165 ml Wasser gewaschen und dann getrocknet, wobei das bei 151° - 152°C schmelzende (±)-III in einer Ausbeute von 53 g (= 95% der Theorie) in Gestalt farbloser Kristalle anfällt.

Beispiel 4

In 2 Litern Dichlormethan suspendiert man 30 g (+)-I-Hydrochlorid und 31,8 g II-Natriumsalz, gibt 500 ml Wasser zu und rührt bei Raumtemperatur für 12 Stunden so, daß eine gute Durchmischung der beiden Phasen erfolgt. Dann trennt man die organische Schicht ab und extrahiert die wässrige Phase noch zweimal mit je 150 ml Dichlormethan. Die vereinigten organischen Schichten werden über Natriumsulfat getrocknet und dann im Vakuum eingeengt. Der schaumige Rückstand wird in etwa 100 ml siedendem Aceton gelöst. Nach dem Abkühlen auf Raumtemperatur fügt man, wenn die Kristallisation beginnt, 100 ml Diethylether zu und bewahrt das Gemisch zur Vervollständigung der Kristallisation im Kühlschrank auf. Der Niederschlag wird abgesaugt und mit Diethylether gewaschen.

Die Mutterlauge wird eingeengt und das Rohprodukt aus Aceton/Diethylether in der beschriebenen Weise umkristallisiert, wobei man weitere 9,5 g des Produktes gewinnt.

Die Gesamtausbeute an (±)-III beträgt 48,5 g (= 87% der Theorie). Das Produkt wird in Form farbloser Kristalle vom Schmelzpunkt 151° - 152°C erhalten.

**Ansprüche**

1) [1e-(m-Methoxyphenyl)-2e-dimethylaminomethyl-cyclohexan-1-(a)-ol]-[ 2-(2',6'-dichloranilino)-phenylacetat] in dem die Base als Razemat oder in optisch aktiver Form vorliegt.

2) (±)-(1e-(m-Methoxyphenyl)-2e-dimethylaminomethyl-cyclohexan-1(a)-ol]-[2-(2',6'-dichloranilino)-phenylacetat].

3) Pharmazeutische Zubereitungen, enthaltend eine Verbindung gemäß Anspruch 1.

4) Pharmazeutische Zubereitungen gemäß Anspruch 3, die zusätzlich zu einer Verbindung gemäß Anspruch 1 noch ein Hydrohalogenid des 1e-(m-Methoxyphenyl)-2e-dimethylaminomethylcyclohexan-1(a)-ol enthalten.

5) Verfahren zur Herstellung einer Verbindung gemäß Anspruch 1, dadurch gekennzeichnet, daß man

a) 1e-(m-Methoxyphenyl)-2e-dimethylaminomethyl-cyclohexan-1(a)-ol (I) als Razemat oder in optisch aktiver Form in Gegenwart eines Lösungsmittels mit einer äquimolaren Menge 2-(2',6'Dichloranilino)-phenylessigsäure (II) neutralisiert oder

b) äquimolare Mengen eines Salzes von I und eines Salzes von II in Gegenwart eines Reaktionsmediums, in dem beide verwendeten Salze löslich sind, umsetzt.

6) Verfahren gemäß Anspruch 5b, dadurch gekennzeichnet, daß ein Hydrohalogenid, vorzugsweise das Hydrochlorid, von I in Gegenwart eines wasserhaltigen Reaktionsmediums mit einem Alkali-oder dem Ammoniumsalz, vorzugsweise dem Natriumsalz, von II umgesetzt wird.

Patentansprüche für folgenden Vertragsstaat : GR

1) [1e-(m-Methoxyphenyl)-2e-dimethylaminomethyl-cyclohexan-1(a)-ol]-[2-(2',6'-dichloranilino)-phenylacetat] in dem die Base als Razemat oder in optisch aktiver Form vorliegt.

2) (±)-[1e-(m-Methoxyphenyl)-2e-dimethylaminomethyl-cyclohexan-1(a)-ol] - [ 2-(2',6'dichloranilino)-phenylacetat.]

3) Verfahren zur Herstellung einer Verbindung gemäß Anspruch 1, dadurch gekennzeichnet, daß man

a) 1e-(m-Methoxyphenyl)-2e-dimethylaminomethyl-cyclohexan-1-(a)-ol (I) als Razemat oder in optisch aktiver Form in Gegenwart eines Lösungsmittels mit einer äquimolaren Menge 2-(2',6'Dichloranilino)-phenylessigsäure (II) neutralisiert oder

b) äquimolare Mengen eines Salzes von I und eines Salzes von II in Gegenwart eines Reaktionsmediums, in dem beide verwendeten Salze löslich sind, umsetzt.

4) Verfahren gemäß Anspruch 3b, dadurch gekennzeichnet, daß ein Hydrohalogenid, vorzugsweise das Hydrochlorid, von I in Gegenwart eines wasserhaltigen Reaktionsmediums mit einem Alkali-oder dem Ammoniumsalz, vorzugsweise dem Natriumsalz, von II umgesetzt wird.

Patentansprüche für folgende Vertragsstaaten : AT ; ES

1) Verfahren zur Herstellung von [1e-(m-Methoxyphenyl)-2e-dimethylaminomethyl-cyclohexan-1(a)-ol]-[2-(2',6'-dichloranilino)-phenylacetat] in dem die Base als Razemat oder in optisch aktiver Form vorliegt, dadurch gekennzeichnet, daß man

a)1e-(m-Methoxyphenyl)-2e-dimethylaminomethyl-cyclohexan-1(a)-ol (I) als Razemat oder in optisch aktiver Form in Gegenwart eines Lösungsmittels mit einer äquimolaren Menge 2-(2',6'Dichloranilino)-phenylessigsäure (II) neutralisiert oder

b) äquimolare Mengen eines Salzes von I und eines Salzes von II in Gegenwart eines Reaktionsmediums, in dem beide verwendeten Salze löslich sind, umsetzt.

2) Verfahren gemäß Anspruch 1, dadurch gekennzeichnet, daß I in Form der freien Base oder eines Salzes als Razemat eingesetzt wird.

3) Verfahren gemäß Ansprüchen 1b und 2 , dadurch gekennzeichnet, daß ein Hydrohalogenid, vorzugsweise das Hydrochlorid, von I in Gegenwart eines wasserhaltigen Reaktionsmediums mit einem Alkali-oder dem Ammoniumsalz, vorzugsweise dem Natriumsalz, von II umgesetzt wird.

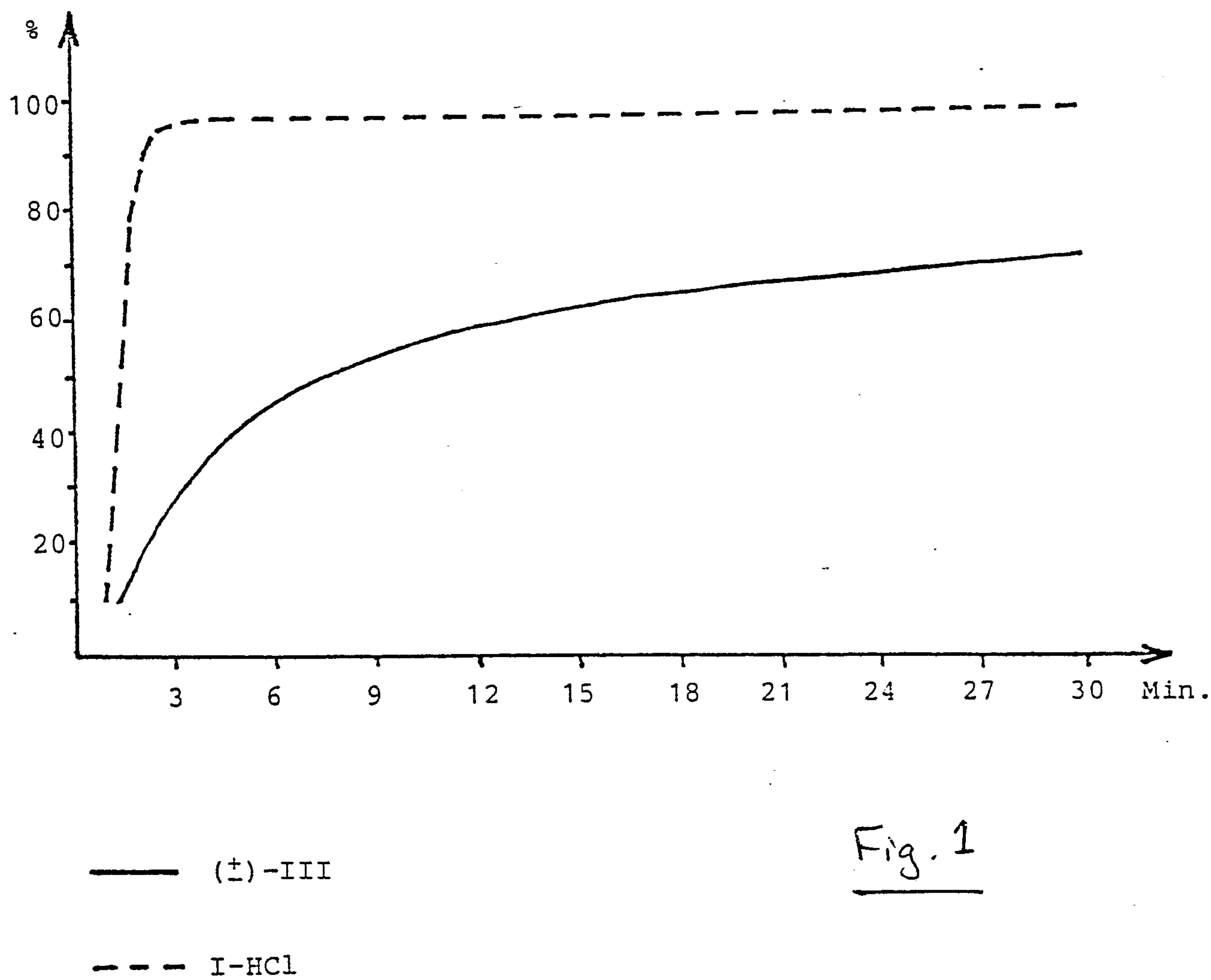


Fig. 1